# EUROPEAN PATENT APPLICATION

(11) **EP 4 477 232 A1**
(43) Date of publication of application: **18.12.2024**
(21) Application number: 23851587.8
(22) Date of filing: 25.07.2023
(51) Int. Cl.: A61K 39/215, A61K 39/295, A61P 31/14, C12N 15/62

(54) **RECOMBIANT MULTIVALENT VACCINE**

(30) Priority: 12.08.2022 CN 202210965587
(71) Applicant: Shanghai Public Health Clinical Center, Shanghai 201599 (CN)
(72) Inventor: YAN, Huimin, Shanghai 201599 (CN); YANG, Jingyi, Shanghai 201599 (CN); CHEN, Yaoqing, Shanghai 201599 (CN); SHI, Zhengli, Shanghai 201599 (CN)
(74) Representative: Bayramoglu et al.
(86) International application number: PCT/CN2023/109037
(87) International publication number: WO 2024/032365

(57) **Abstract**

The present invention relates to a recombinant multivalent vaccine, including a recombinant protein, wherein the recombinant protein includes, from a N-terminus to a C-terminus, a first antigenic peptide, an N-polypeptide (SEQ ID NO. 1), a second antigenic peptide, a C-polypeptide (SEQ ID NO. 3) and a third antigenic peptide, wherein the N-polypeptide and the C-polypeptide are intramolecular scaffold-forming polypeptides, forming an intramolecular scaffold NC for supporting and stabilizing conformations of the first antigenic peptide, the second antigenic peptide and the third antigenic peptide. The present invention provides recombinant multivalent vaccines 3Ro-NC (SEQ ID NO. 17) and 3Rs-NC (SEQ ID NO. 19) against SARS-CoV-2 variants. The present invention uses 3Ro-NC plus KFD as a prophylactic mucosal SARS-CoV-2 vaccine to provide protection against Omicron infection in upper and lower respiratory tracts.

## Description

### TECHNICAL FIELD

The present invention relates to a recombinant multivalent vaccine, in particular to a recombinant multivalent vaccine against cancer and infectious bacteria and viruses, in particular against infectious bacteria and viruses having more than one serological subtype or variant, such as coronaviruses and influenza viruses, and belongs to the technical field of biomedicines.

### BACKGROUND

Cancer results in death; and many cancer-specific or associated antigens have been discovered and have been used to develop cancer vaccines.

Infectious pathogens including bacteria and viruses, especially those with more than one serological subtype or variant, pose a great threat to public health.

A disease caused by SARS-CoV-2 is named COVID-19. Since a SARS-CoV-2 original strain, variants such as Alpha (B.1.1.7), Beta (B. 1.351), Gamma (P.1), Delta (B.1.617.2) and Omicron (B.1.1.529) have emerged one after another, and have caused serious problems about the nature, extent and consequences of antigenic drift in SARS-CoV-2. The unpredictable emergence of SARS-CoV-2 variants of concern (VOC) with different antigenicity has increased the risk of global transmission and led to the COVID-19 pandemic.

In addition to non-pharmaceutical interventions and strict border control measures, several different vaccines based on different platforms, including mRNA, adenovirus and inactivated virus, are deployed clinically worldwide. However, new cases of infection around the world still escalate from time to time.

Therefore, there is an urgent need to explore a new method to develop safe and effective vaccines against cancer and infectious pathogens (such as coronaviruses and influenza viruses), especially effective mucosal vaccines to prevent initial viral infection and potential transmission of a respiratory tract.

### SUMMARY

An object of the present invention is to provide a safe and effective vaccine against cancer and infectious pathogens.

In order to achieve the above object, the technical solution adopted by the present invention is to provide a recombinant multivalent vaccine, including a recombinant protein, wherein the recombinant protein includes, from a N-terminus to a C-terminus, a first antigenic peptide, an N-polypeptide, a second antigenic peptide, a C-polypeptide and a third antigenic peptide;
wherein the N-polypeptide has an amino acid sequence as shown in SEQ ID NO. 1 or a variant thereof;
the C-polypeptide has an amino acid sequence as shown in SEQ ID NO. 3 or a variant thereof; and
the N-polypeptide and the C-polypeptide are intramolecular scaffold-forming polypeptides, forming an intramolecular scaffold NC for supporting and stabilizing conformations of the first antigenic peptide, the second antigenic peptide and the third antigenic peptide.

In certain embodiments of the present invention, the variant of the N-polypeptide is functionally identical to and has at least 95% sequence homology with SEQ ID NO. 1; and the variant of the C-polypeptide is functionally identical to and has at least 95% sequence homology with SEQ ID NO. 3.

In certain embodiments of the present invention, the first antigenic peptide, the second antigenic peptide and the third antigenic peptide are polypeptides consisting of 10-900 amino acids that induce an antigenic peptide specific humoral and/or cellular immune response when the recombinant multivalent vaccine is used to immunize a host subject.

In certain embodiments of the present invention, the first antigenic peptide, the second antigenic peptide and the third antigenic peptide are derived from an infectious pathogen, a variant of an infectious pathogen or a cancer/tumor-specific antigen.

In certain embodiments of the present invention, the first antigenic peptide, the second antigenic peptide and the third antigenic peptide are derived from a SARS-CoV-2 variant.

In certain embodiments of the present invention, the antigenic peptide is a receptor binding domain (RBD) region aa.319-527 of a spike protein of the SARS-CoV-2 variant.

In certain embodiments of the present invention, the RBD is derived from any one of a SARS-CoV-2 original strain, Delta, Omicron, and Gamma variants and SARS-related coronavirus strains such as SHC014 and WIV1, wherein Delta RBD has an amino acid sequence as shown in SEQ ID NO. 5 and a nucleotide sequence as shown in SEQ ID NO. 6, Omicron RBD has an amino acid sequence as shown in SEQ ID NO. 7 and a nucleotide sequence as shown in SEQ ID NO. 8, RBD of the SARS-CoV-2 original strain has an amino acid sequence as shown in SEQ ID NO. 9 and a nucleotide sequence as shown in SEQ ID NO. 10, Gamma RBD has an amino acid sequence as shown in SEQ ID NO. 11 and a nucleotide sequence as shown in SEQ ID NO. 12, SHC014 RBD has an amino acid sequence as shown in SEQ ID NO. 13 and a nucleotide sequence as shown in SEQ ID NO. 14, and WIV1 RBD has an amino acid sequence as shown in SEQ ID NO. 15 and a nucleotide sequence as shown in SEQ ID NO. 16.

In certain embodiments of the present invention, the recombinant protein includes, from the N-terminus to the C-terminus, Omicron RBD, an N-polypeptide, Delta RBD, a C-polypeptide and Omicron RBD, having an amino acid sequence as shown in SEQ ID NO. 17; or the recombinant protein includes, from the N-terminus to the C-terminus, Omicron RBD, an N-polypeptide, SHC014 RBD, a C-polypeptide, and WIV1 RBD, having an amino acid sequence as shown in SEQ ID NO. 19.

In certain embodiments of the present invention, the recombinant multivalent vaccine further includes an adjuvant selected from at least one of flagellin, polyinosinic-polycytidylic acid, MF59, AS01, AS03, AS04, CpG, MPL, CT, CTB, IL-1α, IL-2, IL-12, IL-18, GM-CSF, PIKA, and BFA03.

In certain embodiments of the present invention, the adjuvant is recombinant flagellin KFD, having an amino acid sequence shown in SEQ ID NO. 21 or having at least 95% sequence homology with SEQ ID NO. 21 and retaining the TLR-5 agonist activity.

The present invention also provides a nucleic acid molecule encoding a recombinant protein for a recombinant multivalent vaccine, wherein the nucleic acid molecule includes from 5' to 3': a first polynucleotide encoding a first antigenic peptide, a polynucleotide encoding an N-polypeptide, a second polynucleotide encoding a second antigenic peptide, a polynucleotide encoding a C-polypeptide, and a third polynucleotide encoding a third antigenic peptide;
wherein the polynucleotide encoding the N-polypeptide has a nucleotide sequence shown in SEQ ID NO. 2 or has at least 95% sequence homology with SEQ ID NO. 2;
the polynucleotide encoding the C-polypeptide has a nucleotide sequence shown in SEQ ID NO. 4 or has at least 95% sequence homology with SEQ ID NO. 4; and
when a recombinant protein encoded by the nucleic acid molecule is expressed, the N-polypeptide and the C-polypeptide are intramolecular scaffold-forming polypeptides, forming an intramolecular scaffold NC for supporting the first antigenic peptide, the second antigenic peptide and the third antigenic peptide.

Compared with the prior art, the present invention has the following beneficial effects:
(1) the present invention provides the recombinant multivalent vaccine based on the intramolecular scaffold that can maintain the conformations of the antigenic peptides, and the antigenic peptides are presented at three different positions of the intramolecular scaffold; the intramolecular scaffold is applicable to any antigenic peptide, whether it is of viral, bacterial or cancer origin; thus, the present invention solves at least two problems in vaccine development: first, the conformations of the antigenic peptides are maintained, which ensures that the antigenic peptides can induce a humoral and cellular immune response, especially effective neutralizing antibody responses; and second, presentation of multivalent antigenic peptides can induce humoral and cellular immune responses against more than one cancer or more than one infectious pathogen variant;
(2) further, the present invention provides a recombinant multivalent vaccine against SARS-CoV-2. Immunogens of chimeric RBD-dimers with tandem RBDs are designed by using RBDs of different strains, and SARS-CoV-2 RBD conjugated with an Fc fragment of human IgG as an immune enhancer can induce strong antibody neutralizing activity against SARS-CoV-2 infection in mice, overcoming the limitations of immunogenicity of viral RBD antigens and inducing broad-spectrum immune responses, and overcoming the deficiency that RBD-based subunit vaccines do not strongly induce mucosal immune responses in the respiratory tract to prevent SARS-CoV-2 nasal infection and asymptomatic transmission;
(3) the recombinant multivalent vaccine against SARS-CoV-2 provided by the present invention includes a recombinant protein 3Ro-NC or 3Rs-NC having a natural conformation of RBD, thus having strong immunogenicity, and can effectively induce a broad-spectrum protective immune response against SARS-CoV-1 and SARS-CoV-2 variants, particularly against the immune escape Omicron variant; and
(4) further, the recombinant multivalent vaccine against SARS-CoV-2 provided by the present invention includes a mucosal adjuvant recombinant flagellin KFD; intranasal immunization with 3Ro-NC plus recombinant flagellin KFD as the mucosal adjuvant can induce synergistic mucosal and systemic immunity against SARS-CoV-1 and SARS-CoV-2 variants in mice; antibodies induced by intranasal immunization with 3Ro-NC plus KFD show higher specificity and neutralization to the Omicron variant; in human ACE2 transgenic mice infected with the Omicron variant, immunity induced by intranasal immunization with 3Ro-NC plus KFD significantly reduces the copy number of viral RNAs in the lungs and nasal turbinates; and in addition, a significant reduction in histopathology is detected in the lungs of nasally inoculated mice.

### BRIEF DESCRIPTION OF THE DRAWINGS

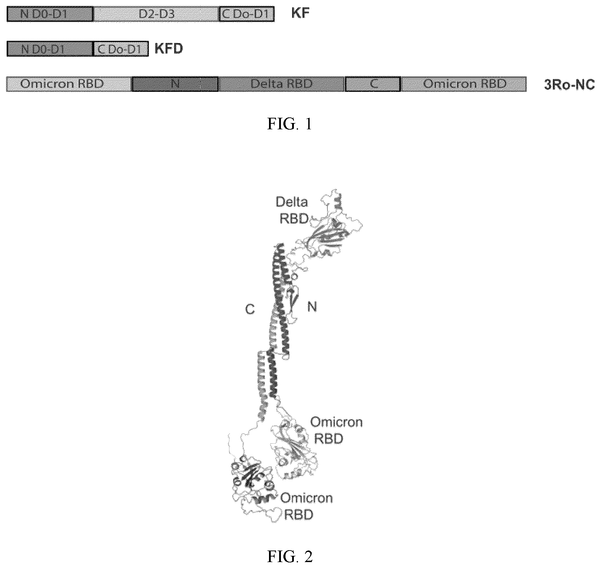

FIG. 1 is a schematic domain configuration of KF, KFD and a chimeric triple RBD protein called 3Ro-NC;
FIG. 2 is a schematic diagram of a 3D structure of 3Ro-NC predicted by Alpha Fold 2;

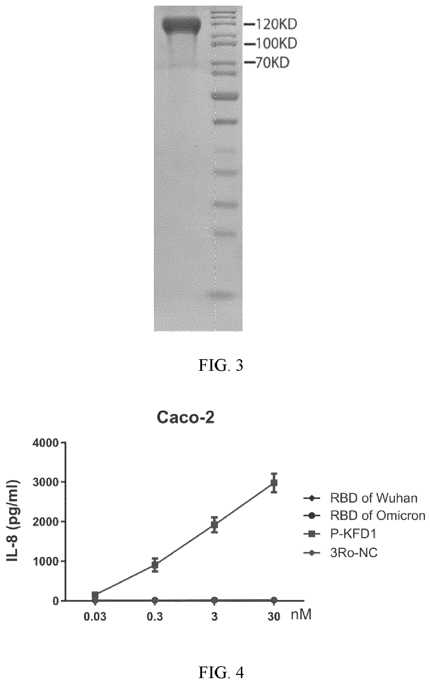

FIG. 3 is a Western blot image of purified 3Ro-NC;
FIG. 4 shows the TLR5 agonist activity of recombinant proteins such as RBD of a SARS-CoV-2 original strain, RBD of an Omicron strain, P-KFD, and 3Ro-NC;

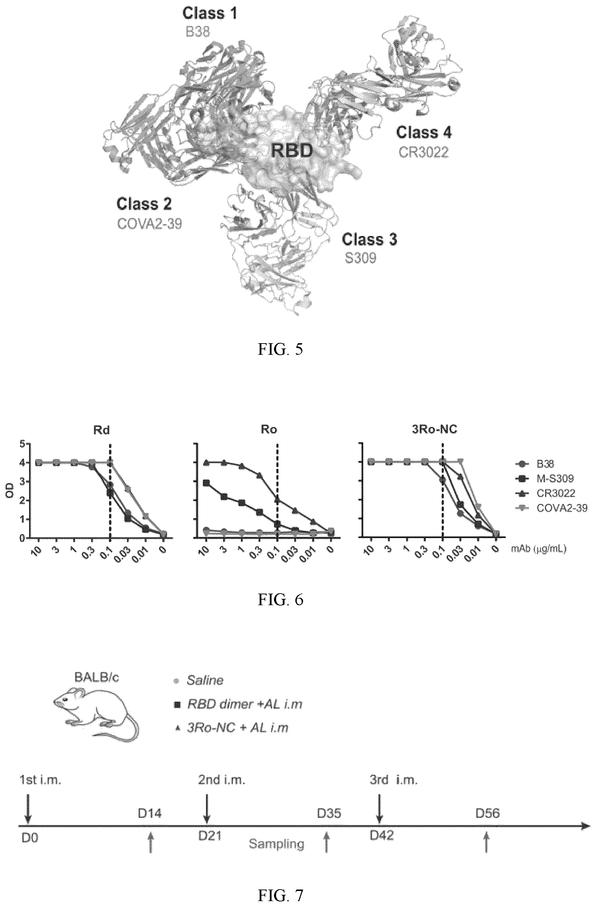

FIG. 5 is a 3D schematic diagram of binding of SARS-CoV-2 RBD to four classes of neutralizing monoclonal antibodies B38, M-S309, CR3022, and COVA2-39;
FIG. 6 is a graph showing the results of ELISA detection of the binding ability of RBD of a Delta strain (Rd), RBD of an Omicron strain (B. 1.1.529) (Ro), and 3Ro-NC to four representative classes of neutralizing monoclonal antibodies B38, M-S309, CR3022, and COVA2-39;
FIG. 7 is a schematic diagram of an immunization and sampling schedule (5 mice per group);

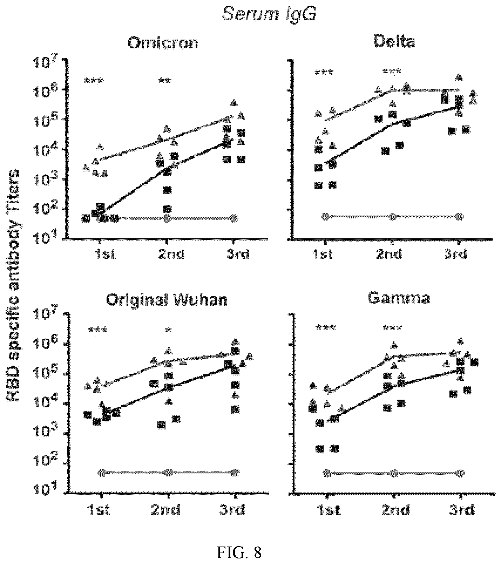

FIG. 8 is a graph showing RBD-specific IgG responses in serum, wherein filled triangles represent a 3Ro-NC group, filled squares represent an RBD-dimer group, and filled circles represent a control; data are expressed as mean±SEM and are representative of at least two independent experiments; two immunization groups are compared by using an unpaired t-test; *p<0.05; **p<0.01; and ***p<0.001; ns: not significant;

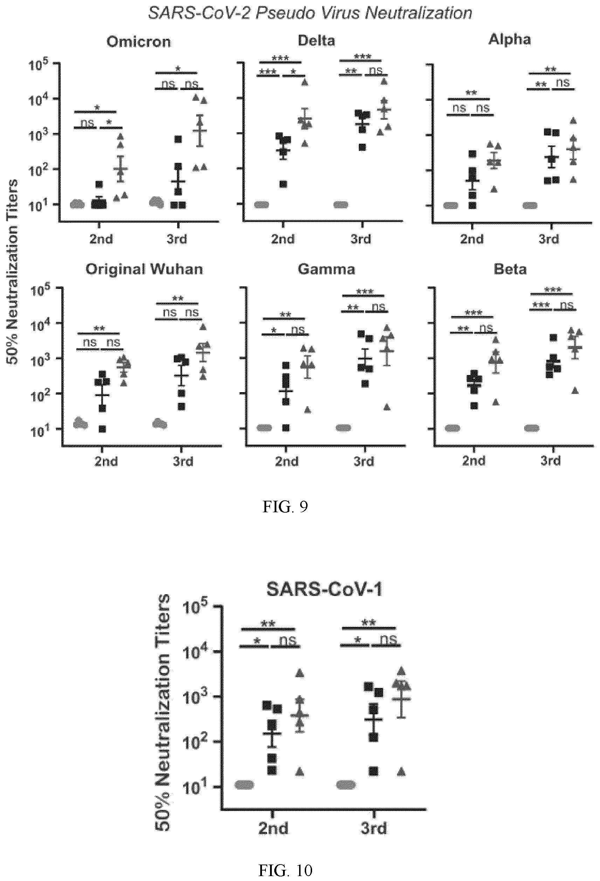

FIG. 9 is a dot plot showing the results of titers of neutralizing antibodies against SARS-CoV-2 variants after the 2nd and 3rd immunizations in a pseudovirus assay, wherein filled triangles represent the 3Ro-NC group, filled squares represent the RBD dimer group, and filled circles represent the control; data are expressed as mean±SEM and are representative of at least two independent experiments; the groups are compared by using one-way ANOVA; *p<0.05; **p<0.01; and ***p<0.001; ns: not significant;
FIG. 10 is a dot plot showing the results of titers of neutralizing antibodies against SARS-CoV-1 after the 2nd and 3rd immunizations in a pseudovirus assay, wherein filled triangles represent the 3Ro-NC group, filled squares represent the RBD dimer group, and filled circles represent the control; data are expressed as mean±SEM and are representative of at least two independent experiments; the groups are compared by using one-way ANOVA; *p<0.05; **p<0.01; and ***p<0.001; ns: not significant;

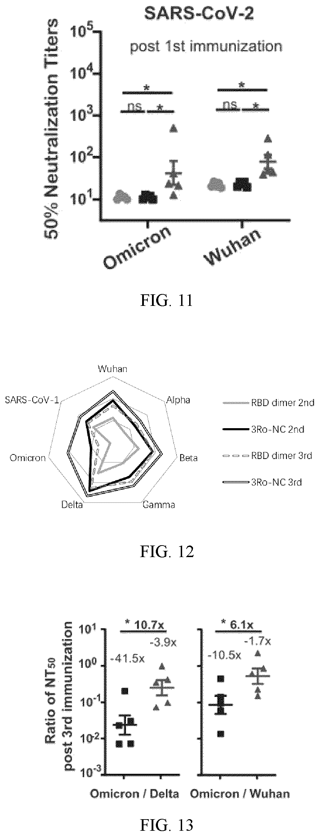

FIG. 11 is a dot plot showing the results of titers of neutralizing antibodies against Omicron and the original strain after the 1st immunization in a pseudovirus assay, wherein filled triangles represent the 3Ro-NC group, filled squares represent the RBD dimer group, and filled circles represent the control; data are expressed as mean±SEM and are representative of at least two independent experiments; the groups are compared by using one-way ANOVA; *p<0.05; **p<0.01; and ***p<0.001; ns: not significant;
FIG. 12 is a graph showing a geometric mean titer (GMT) of neutralizing antibodies against different variants;
FIG. 13 is a dot plot showing a ratio of the titer (a 50% neutralization titer, NT50) of neutralizing antibodies against an Omicron strain to the titer of neutralizing antibodies against a SARS-CoV-2 original strain or a Delta strain, wherein data are expressed as mean±SEM and are representative of at least two independent experiments; two immunization groups are compared by using an unpaired t-test; *p<0.05; **p<0.01; and ***p<0.001; ns: not significant;

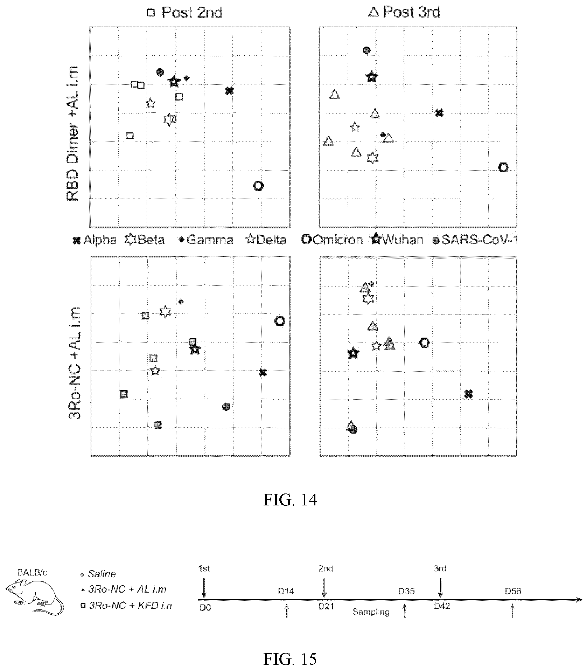

FIG. 14 is an antigenic map generated from serum samples post 2nd (squares) or 3rd (triangles) vaccination in RBD dimer (white) and 3Ro-NC (grey) groups, wherein ×, 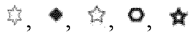 , and ● correspond to Alpha, Beta, Gamma, Delta, Omicron, a SARS-CoV-2 original strain and SARS-CoV-1, respectively; and each square corresponds to a two-fold dilution in a neutralization assay;
FIG. 15 is a schematic diagram of an immunization and sampling schedule in which antibody responses are generated by intranasal immunization with 3Ro-NC and KFD as an adjuvant in BALB/c mice (5 mice/group);

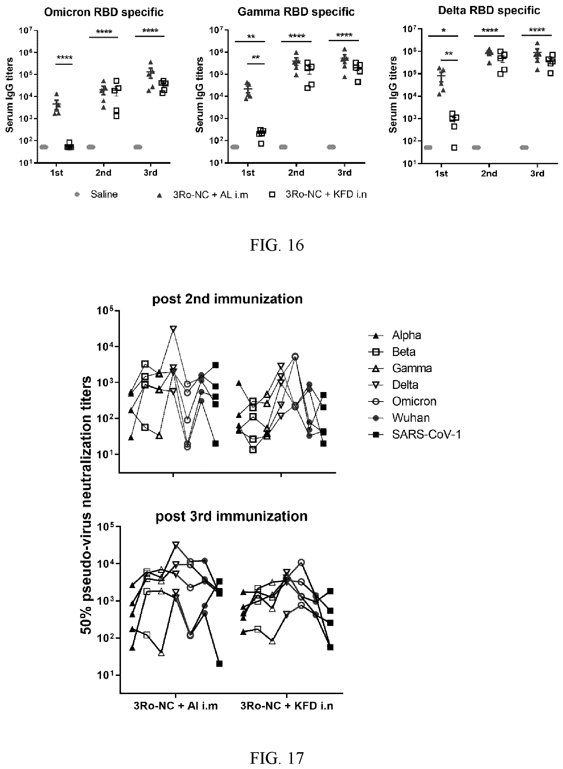

FIG. 16 is a dot plot showing the results of Delta, Gamma or Omicron RBD-specific IgG responses in serum, wherein data are expressed as mean±SEM and are representative of at least two independent experiments; differences between the groups are compared by using one-way ANOVA; *p<0.05; **p<0.01; and ***p<0.001; ns: not significant;
FIG. 17 is a graph showing titers of neutralizing antibodies against SARS-CoV-2 variants or SARS-CoV-1 by a pseudovirus assay in serum post 2nd and 3rd immunizations in 3Ro-NC+KFD intranasal immunization and 3Ro-NC+AL intramuscular injection groups, wherein each connecting line represents a neutralization titer of one single serum sample to a different viral strain;

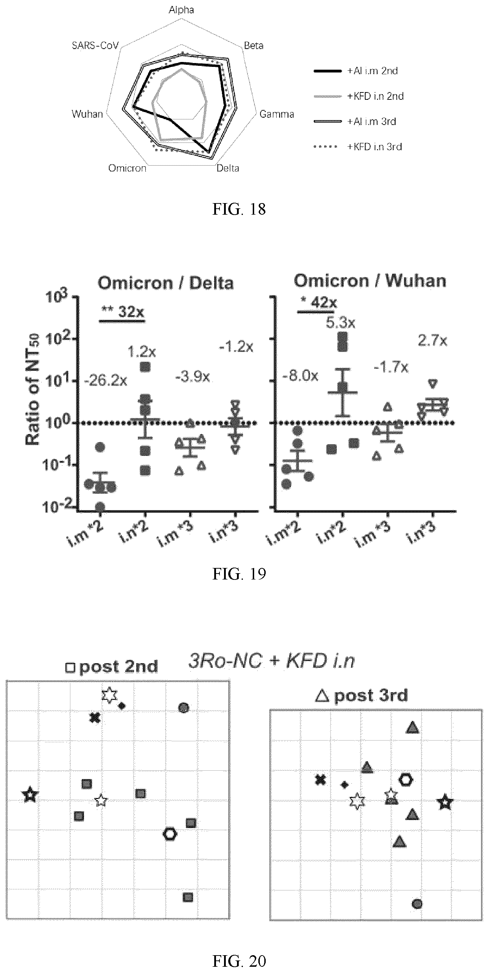

FIG. 18 is a graph showing a geometric mean titer of neutralizing antibodies against different variants in serum post 2nd and 3rd immunizations in 3Ro-NC+KFD intranasal immunization and 3Ro-NC+AL intramuscular injection groups;
FIG. 19 is a dot plot showing a ratio of the titer of neutralizing antibodies against Omicron to the titer of neutralizing antibodies against Delta or a SARS-CoV-2 original strain, wherein data are expressed as mean±SEM and are representative of at least two independent experiments; differences between the groups are compared by using one-way ANOVA; *p<0.05; **p<0.01; and ***p<0.001; ns: not significant;
FIG. 20 is an antigenic map generated from serum samples post 2nd (squares) or 3rd (triangles) vaccination in a 3Ro-NC+KFD intranasal immunization group, wherein ×, 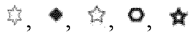 , and ● correspond to Alpha, Beta, Gamma, Delta, Omicron, a SARS-CoV-2 original strain and SARS-CoV-1, respectively; each square corresponds to a two-fold dilution in a neutralization assay; and an antigenic distance can be interpreted in any direction;

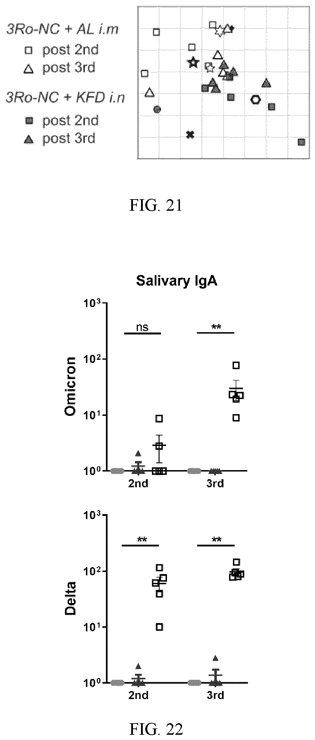

FIG. 21 is an antigenic map generated by a 3Ro-NC+KFD intranasal immunization group (brown) and a 3Ro-NC+AL intramuscular injection group (gray), wherein ×, 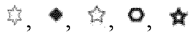 , and ● correspond to Alpha, Beta, Gamma, Delta, Omicron, a SARS-CoV-2 original strain and SARS-CoV-1, respectively; each square corresponds to a two-fold dilution in a neutralization assay; and an antigen distance can be interpreted in any direction;
FIG. 22, FIG. 23 and FIG. 24 are dot plots showing RBD-specific mucosal IgA responses in saliva, vaginal lavage fluid and nasal turbinate lavage fluid, respectively, wherein data are expressed as mean±SEM and are representative of at least two independent experiments. Differences between the groups are compared by using one-way ANOVA; *p<0.05; **p<0.01; and ***p<0.001; ns, not significant;

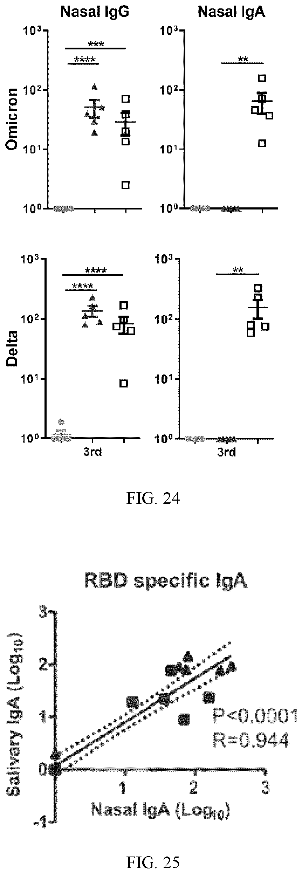

FIG. 25, FIG. 26 and FIG. 27 show the correlation between RBD-specific IgA in nasal lavage fluid and RBD-specific IgA in saliva (triangles represent Delta; and squares represent Omicron), the correlation between RBD-specific IgG in nasal lavage fluid and RBD-specific IgG in serum (triangles represent Delta; and squares represent Omicron), and the correlation between RBD-specific IgA and IgG in nasal lavage fluid and neutralizing antibody responses of BALB/c mice immunized with 3Ro-NC determined in a pseudovirus system, respectively;

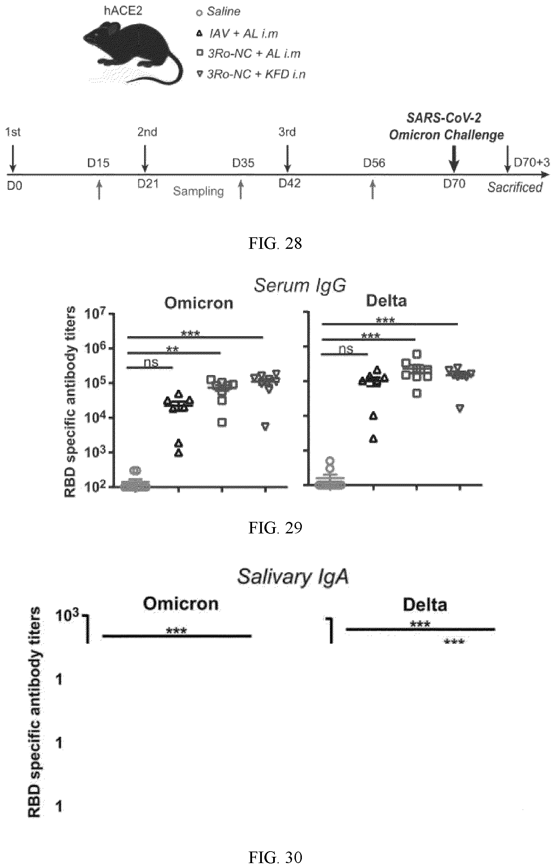

FIG. 28 shows a schematic diagram of an immunization and viral challenge schedule used to investigate protection of 3Ro-NC immunized hACE2 mice against infection with an Omicron variant of SARS-CoV-2 (6-8 mice per group);
FIG. 29, FIG. 30 and FIG. 31 are dot plots showing Omicron, Delta and Gamma RBD-specific serum IgG titers; Delta and Omicron RBD-specific salivary IgA; and Delta and Omicron RBD-specific vaginal lavage fluid IgA post 3rd immunization, respectively, wherein shape symbols used in each group have the same meaning as those in FIG. 28; the groups are compared by using one-way ANOVA; *p<0.05; **p<0.01; and ***p<0.001; ns: not significant;

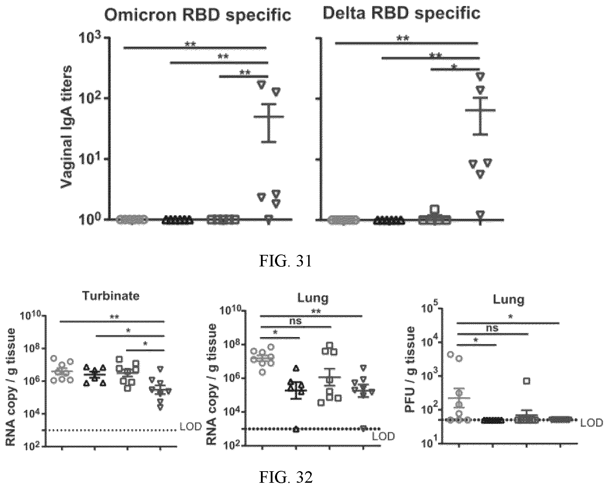

FIG. 32 is a dot plot showing the RNA copy number of SARS-CoV-2 RBD in nasal turbinates and lungs tested by qPCR, and a plaque assay of infectious virus in lungs 3 days post-infection, respectively, wherein differences between the groups are compared by using one-way ANOVA; *p<0.05; **p<0.01; and ***p<0.001; ns: not significant;

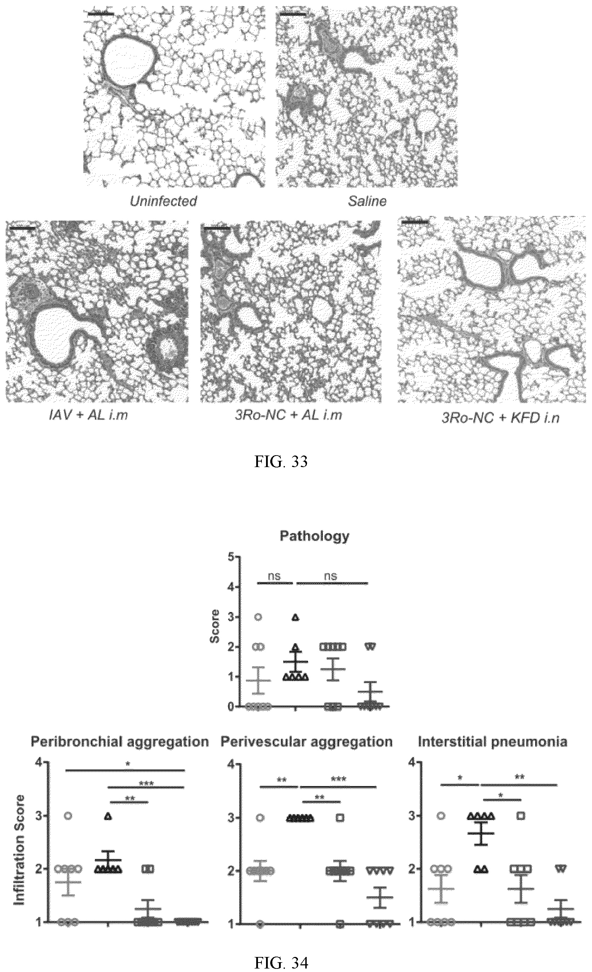

FIG. 33 shows images of hematoxylin and eosin (H&E) staining of lung sections (scale, 100 µm);
FIG. 34 is a dot plot showing pathology scores and infiltration scores of immune cell aggregation around bronchioles, pulmonary vessels and interstitial pneumonia according to H&E stained sections (6-8 mice/group), wherein shape symbols used in each group has the same meaning as those in FIG. 28; the groups are compared by using one-way ANOVA; *p<0.05; **p<0.01; and ***p<0.001; ns: not significant;

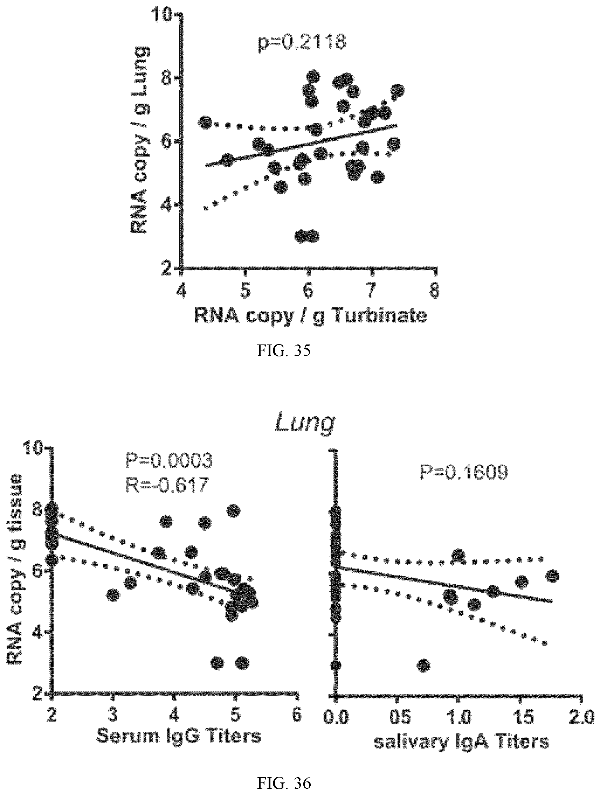

FIG. 35 is a graph showing the correlation between the RNA copy number in lungs and nasal turbinates, wherein a 95% confidence interval is indicated by a dashed line; differences between the groups are compared by using one-way ANOVA; *p<0.05; **p<0.01; and ***p<0.001; ns: not significant;

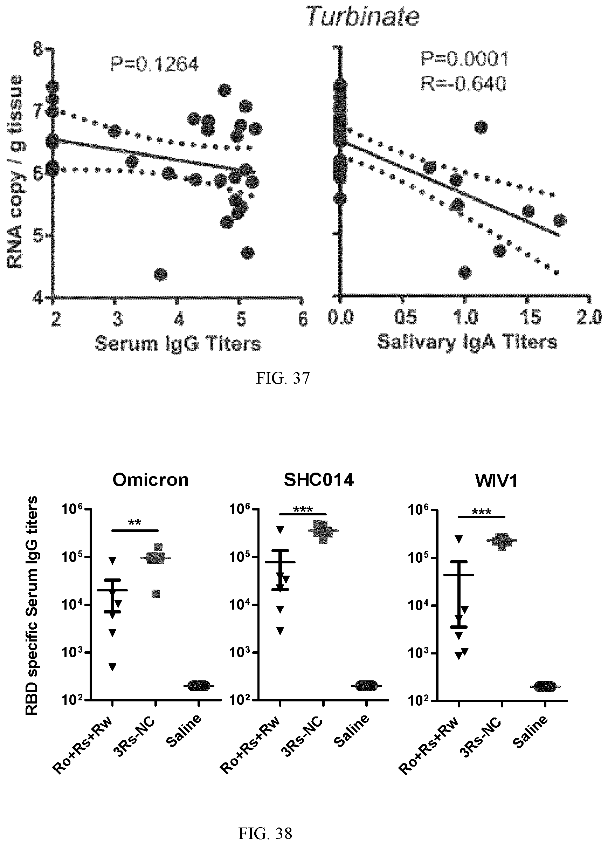

FIGS. 36 and 37 are graphs showing the correlation between the RNA copy number in lungs and nasal turbinates and Omicron RBD-specific serum IgG and salivary IgA, respectively, wherein a 95% confidence interval is indicated by a dashed line; differences between the groups are compared by using one-way ANOVA; *p<0.05; **p<0.01; and ***p<0.001; ns: not significant;
FIG. 38 is a dot plot showing serum RBD-specific IgG responses post 2nd immunization, wherein triangles represent an Ro+Rs+Rw group (1.33 µg Ro (Omicron RBD)+1.33 µg Rs (SHC014 RBD)+1.33 µg Rw (WIV1 RBD) plus 200 µg an aluminum adjuvant), squares represent a 4 µg 3Rs-NC group plus 200 µg an aluminum adjuvant, and circles represent the control group; data are expressed as mean±SEM and are representative of at least two independent experiments; two immunization groups are compared by using an unpaired t-test; **p<0.01; and ***p<0.001;

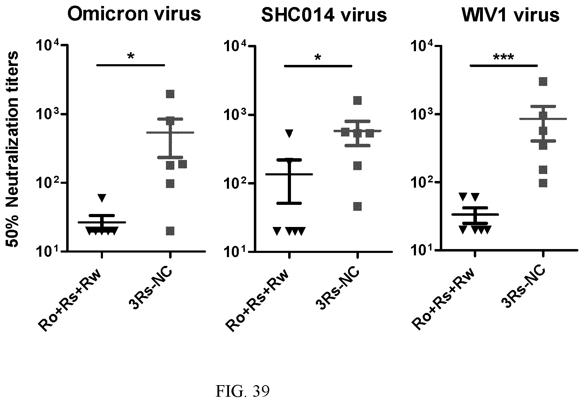

FIG. 39 is a dot plot showing titers of neutralizing antibodies against a SARS-CoV-2 variant Omicron or SARS-like viruses SHCO14 and WIV1 post 2nd immunization, wherein data are expressed as mean±SEM; the groups are compared by using one-way ANOVA; *p<0.05; *** p<0.001.

### DETAILED DESCRIPTION

In order to make the present invention more obvious and understandable, preferred examples are described in detail below with reference to the accompanying drawings.

Unless otherwise indicated, conventional techniques of molecular biology (including recombinant techniques), microbiology, cell biology, biochemistry, nucleic acid chemistry and immunology, which are within the skill of the art will be employed in the practice of the present invention. These techniques have been explained fully in the literature, such as Molecular Cloning: A Laboratory Manual, Third Edition (Sambrook and Russel, 2001); and Current Protocols in Molecular Biology (edited by FM Ausubel et al., 1988). In the present invention, "cancer" is a collective term for any type or form of tumor and cancer.

Many types of vaccines have been developed against COVID-19, such as live attenuated virus, inactivated virus virion or subunit vaccines. Recombinant protein subunit vaccines containing viral purified proteins are the safest type of immunogen for vaccine development. Such subunit vaccines do not cause COVID-19 because they contain only purified viral protein fragments. Protein subunit vaccines have been used for other diseases such as hepatitis B (HBV) and cervical cancer (HPV). However, how to maintain a native conformation of recombinant subunit proteins in subunit vaccines has been a grave challenge. Current approaches include human Fc peptides, a cloverleaf structure technology, self-assembling nanoparticles (such as ferritin), and the like.

The present invention addresses at least two problems in vaccine development. First, maintenance of the conformation of antigenic peptides; this ensures that the antigenic peptides can induce a humoral and cellular immune response, especially the effective neutralizing antibody responses; and second, presentation of multivalent antigenic peptides, this enables to induce humoral and cellular immune responses against more than one cancer or more than one infectious pathogen variant. The present invention provides an intramolecular scaffold that can maintain the conformations of the antigenic peptides, and the antigenic peptides are presented at three different positions of the intramolecular scaffold. The intramolecular scaffold is applicable to any antigenic peptide, whether it is derived from viruses, bacteria or cancer. A multivalent vaccine of COVID-19 described hereinafter fully illustrates the practicality of the present invention.

The present invention provides a recombinant multivalent vaccine including a recombinant protein, wherein the recombinant protein includes, from a N-terminus to a C-terminus, a first antigenic peptide, an N-polypeptide, a second antigenic peptide, a C-polypeptide and a third antigenic peptide, wherein the N-polypeptide and the C-polypeptide are intramolecular scaffold-forming polypeptides, forming an intramolecular scaffold NC for stabilizing conformations of the first antigenic peptide, the second antigenic peptide and the third antigenic peptide. In certain embodiments, a linker is inserted at one or more of the junctions between the first antigenic peptide and the N-polypeptide, between the N-polypeptide and the second antigenic peptide, between the second antigenic peptide and the C-polypeptide, and between the C-polypeptide and the third antigenic peptide. The linker may consist of 4-40 amino acids; and an exemplary linker is (GSSS)n, wherein n is 1-10.

The N-polypeptide and the C-polypeptide of the intramolecular scaffold NC are developed via the modification of flagellin derived from an Escherichia coli K12 strain (KF). In certain embodiments, the N-polypeptide of the intramolecular scaffold NC has an amino acid sequence as shown in SEQ ID NO. 1 or a variant thereof, wherein the variant has at least 95% sequence homology with SEQ ID NO. 1. In certain embodiments, the N-polypeptide of the intramolecular scaffold NC is encoded by a nucleotide sequence as shown in SEQ ID NO. 2 or a variant thereof, wherein the variant has at least 95% sequence homology with SEQ ID NO. 2. In certain embodiments, the C-polypeptide of the intramolecular scaffold NC has an amino acid sequence as shown in SEQ ID NO. 3, or a variant thereof, wherein the variant has at least 95% sequence homology with SEQ ID NO. 3. In certain embodiments, the C-polypeptide of the intramolecular scaffold NC is encoded by a nucleotide sequence as shown in SEQ ID NO. 4 or a variant thereof, wherein the variant has at least 95% sequence homology with SEQ ID NO. 4.

The antigenic peptide can be a polypeptide that can induce a humoral B cell epitope-based and/or cellular T cell epitope-based immune response when the recombinant multivalent vaccine is used to immunize a host subject including, for example, humans, cows, cats, dogs, horses, and pigs. In certain embodiments, the first antigenic peptide, the second antigenic peptide and the third antigenic peptide are all derived from one variant of one infectious pathogen or derived from one cancer-specific antigen. In certain embodiments, the first antigenic peptide, the second antigenic peptide and the third antigenic peptide are derived from one or more variants of one infectious pathogen, or one or more cancer-specific antigens of one cancer. In certain embodiments, the antigenic peptides are derived from one or more infectious pathogens, or derived from one or more cancers. Tumor-specific antigens include, for example, MARA antigens in melanoma, and Ras oncogenic mutants in gastrointestinal and lung cancers. The "cancer-specific antigen" in the present invention includes cancer/tumor-specific antigens, cancer/tumor-associated antigens, and cancer-associated pathogens; for example, the tumor-specific antigens include MARA antigens in melanoma, and Ras oncogenic mutants in gastrointestinal and lung cancers, the cancer/tumor-associated antigens include CD19 and CD20, and the cancer-associated pathogens include Epstein-Barr virus, HPV and HBV, and the like. The antigenic peptides contain 10-900, 10-600, or 10-300 amino acids.

The present invention provides a recombinant multivalent vaccine against SARS-CoV-2 variants.

According to clinical findings, infection and replication of SARS-CoV-2 typically begin in nasal ciliated cells. However, the nasal ciliated cells are hardly accessible to the serum IgG antibodies that are typically induced by vaccinated COVID-19 vaccines, resulting in low efficiency in preventing initial viral entry and infection of the upper respiratory tract. A highly infectious variant Omicron is vaccine-escaping and causes breakthrough infections. Furthermore, more than 90% of Omicron infections are asymptomatic or mild cases, but actually harbor replicating SARS-CoV-2 virus in the nasopharyngeal mucosa and communicate the replicating SARS-CoV-2 virus to others.

The spike (S) protein on the surface of the SARS-CoV-2 virus mediates virus attachment and entry via a receptor binding domain (RBD) (aa. 319-527), which makes it a potential target for COVID-19 vaccine design. RBD-based antigens have been widely used in the development of coronavirus vaccines. However, the protection rate of SARS-CoV-2 vaccines declined with the emergence of COVID-19 variants. To overcome the limitations of immunogenicity of viral RBD antigens and induce broad-spectrum immune responses, immunogens of chimeric RBD-dimers with tandem RBDs are designed by using RBDs of different strains. SARS-CoV-2 RBD conjugated with an Fc fragment of human IgG as an immune enhancer can induce strong antibody neutralizing activity against SARS-CoV-2 infection in mice. However, these RBD-based subunit vaccines do not strongly induce mucosal immune responses in the respiratory tract to prevent SARS-CoV-2 nasal infection and asymptomatic transmission.

In certain embodiments, the recombinant multivalent vaccine against SARS-CoV-2 variants includes a recombinant protein, wherein the recombinant protein includes, from the N-terminus to the C-terminus, a first RBD, an N-polypeptide, a second RBD, a C-polypeptide, and a third RBD, wherein the N-polypeptide and the C-polypeptide have been described above. In certain embodiments, the first RBD, the second RBD, and the third RBD are derived from one SARS-CoV-2 variant. In certain embodiments, the first RBD, the second RBD, and the third RBD are derived from one or more SARS-CoV-2 variants.

In certain embodiments, the RBD is derived from a SARS-CoV-2 original strain, Delta, Omicron, and Gamma variants and SARS-related coronavirus strains SHC014 and WIV1. Delta RBD has an amino acid sequence as shown in SEQ ID NO. 5 and a corresponding nucleotide sequence shown in SEQ ID NO. 6. Omicron RBD has an amino acid sequence as shown in SEQ ID NO. 7 and a corresponding nucleotide sequence shown in SEQ ID NO. 8. RBD of the SARS-CoV-2 original strain has an amino acid sequence as shown in SEQ ID NO. 9 and a corresponding nucleotide sequence shown in SEQ ID NO. 10. Gamma RBD has an amino acid sequence as shown in SEQ ID NO. 11 and a corresponding nucleotide sequence shown in SEQ ID NO. 12. SHC014 RBD has an amino acid sequence as shown in SEQ ID NO. 13 and a corresponding nucleotide sequence shown in SEQ ID NO. 14. WIV1 RBD has an amino acid sequence as shown in SEQ ID NO. 15 and a corresponding nucleotide sequence shown in SEQ ID NO. 16.

In certain embodiments, the recombinant multivalent vaccine against SARS-CoV-2 variants includes a recombinant protein, wherein the recombinant protein includes, from the N-terminus to the C-terminus, Omicron RBD, an N-polypeptide, Delta RBD, a C-polypeptide, and Omicron RBD (designated as 3Ro-NC). 3Ro-NC is highly immunogenic with a native conformation of RBD and potent to induce broad-spectrum protective immune responses against SARS-CoV-1 and SARS-CoV-2 variants, particularly against the immune escape Omicron variant. The 3Ro-NC used for the experiments performed in the embodiments has an amino acid sequence (SEQ ID NO. 17) and a nucleotide sequence (SEQ ID NO. 18).

In certain embodiments, the recombinant multivalent vaccine against SARS-CoV-2 variants includes a recombinant protein, wherein the recombinant protein includes, from the N-terminus to the C-terminus, Omicron RBD, an N-polypeptide, SHC014 RBD, a C-polypeptide, and WIV1 RBD (designated as 3Rs-NC). 3Rs-NC is highly immunogenic with a native conformation of RBD and potent to induce broad-spectrum protective immune responses against the SARS-CoV-1 and SARS-CoV-2 variants. The 3Rs-NC used for the experiments performed in the embodiments has an amino acid sequence (SEQ ID NO. 19) and a nucleotide sequence (SEQ ID NO. 20).

In certain embodiments, the recombinant multivalent vaccine further includes an adjuvant. The adjuvant includes pathogen-associated molecular patterns such as flagellin, poly I:C, MF59 (Novartis), AS series such as AS01, AS03, and AS04 (GSK), Matrix-M (Novavax), CpG (e.g., CpG1018) (Dynavax), MPL (Lipid A-based), cytokines (e.g., IL-2, IL-12, and GM-CSF), PIKA (YISHENG BIOPHARMA), and BFA03 (Jiangsu Recbio).

In certain embodiments, a mucosal adjuvant includes CT, CTB, IL-1α, IL-12, IL-18, CpG (e.g., CpG1018) (Dynavax), cytokines (e.g., IL-2, IL-12, and GM-CSF), PIKA (YISHENG BIOPHARMA), and flagellin.

In certain embodiments, the mucosal adjuvant is recombinant flagellin KFD, which is proven to be safe and effective for intranasal immunization in the inventors' previous works. The KFD has an amino acid sequence shown in SEQ ID NO. 21 or its variants, wherein the variants have at least 95% sequence homology with SEQ ID NO. 21, and a nucleotide sequence shown in SEQ ID NO. 22 or its variants, wherein the variants have at least 95% sequence homology with SEQ ID NO. 22.

The KFD and its variants exert their immunomodulatory activity by activating a TLR5 pathway in nasal epithelial cells and enhancing local and distal mucosal IgA responses. Intranasal immunization with 3Ro-NC plus recombinant flagellin KFD as the mucosal adjuvant can induce synergistic mucosal and systemic immunity against SARS-CoV-1 and SARS-CoV-2 variants in mice. Notably, antibodies induced by intranasal immunization with 3Ro-NC plus KFD show higher specificity and neutralization to the Omicron variant compared with intramuscular injection immunization with either 3Ro-NC plus an aluminum adjuvant or RBD-dimer plus an aluminum adjuvant. Immunity induced by intranasal immunization with 3Ro-NC plus KFD significantly reduces the copy number of viral RNAs in the lungs and the nasal turbinates of the Omicron variant-infected human ACE2 transgenic mice. In addition, a significant reduction in histopathology is detected in the lungs of the intranasally vaccinated mice. Correlation analysis indicates that the degree of nasal virus control is highly correlated with the level of response to RBD-specific secretory IgA antibodies.

The amount of the recombinant protein for inducing a sufficient immune response against an infectious pathogen or cancer in the recombinant multivalent vaccine can be determined by routine clinical trials. In certain embodiments, the recombinant protein in a dose of vaccine is in the range of 1-1000 µg, 10-500 µg, or 50-250 µg.

The present invention also provides a nucleic acid molecule encoding a recombinant protein for a recombinant multivalent vaccine, wherein the nucleic acid molecule includes from 5' to 3': a first polynucleotide encoding a first antigenic peptide, a polynucleotide encoding an N-polypeptide, wherein the polynucleotide encoding the N-polypeptide has a nucleotide sequence shown in SEQ ID NO. 2 or its variant, a second polynucleotide encoding a second antigenic peptide, a polynucleotide encoding a C-polypeptide, wherein the polynucleotide encoding the C-polypeptide has a nucleotide sequence shown in SEQ ID NO. 4 or its variant, and a third polynucleotide encoding a third antigenic peptide, wherein when a recombinant protein encoded by the nucleic acid molecule is expressed, the N-polypeptide and the C-polypeptide are intramolecular scaffold-forming polypeptides, forming an intramolecular scaffold NC for supporting the antigenic peptides. The "variant" means having at least 95% sequence homology with SEQ ID NO. 2 or 4. In certain embodiments, the antigenic peptide consists of 10-900 amino acids. In certain embodiments, the antigenic peptide is a receptor binding domain (RBD) region (aa. 319-527) of the spike (S) protein of SARS-CoV-2 variants. In certain embodiments, the RBD region is a polypeptide represented by SEQ ID NO. 5 (Delta), SEQ ID NO. 7 (Omicron), SEQ ID NO. 9 (the SARS-CoV-2 original strain), SEQ ID NO. 11 (Gamma), SEQ ID NO. 13 (SHC014), and SEQ ID NO. 15 (WIV1).

The nucleic acid molecule may be present in mRNA, an expression vector, or a viral vector (e.g., adenoviruses and adeno-associated viruses). The number of nucleic acid molecules that can induce a sufficient immune response to an infectious pathogen or cancer can be determined by routine clinical studies.

The recombinant multivalent vaccine further includes any pharmaceutically acceptable components, such as excipients (e.g., a saline solution, PBS, etc.).

The following embodiments are provided for the sole purpose of illustrating the principles of the present invention and by no means intended to limit the scope of the present invention.

### Embodiments

### 1. Materials and methods

### 1.1. Mice and ethics

Female 6-to 8-week-old BALB/c mice were purchased from Beijing Vital River Laboratory Animal Technology Co., Ltd. (Beijing, China). HFH4-hACE2 transgenic mice on C57BL/6 background were obtained from Dr. Ralph Baric at the University of North Carolina at Chapel Hill, bred and housed at the Wuhan Institute of Virology (WIV) Animal Center, Chinese Academy of Sciences (CAS). The mice were randomly divided into groups. All mice were housed in individually ventilated cages (IVCs) under specific pathogen-free (SPF) conditions. The infection experiments were performed at the Animal Biosafety Level 3 (ABSL-3) laboratory at WIV, CAS. Animal studies were approved by the Animal Welfare and Ethics Review Committee of Wuhan Institute of Virology in accordance with the Regulations for the Administration of Affairs Concerning Experimental Animals in China (Study No. WIVA09202101).

### 1.2. Vaccine preparation

The N-terminal and C-terminal regions of D0-D1 genes of flagellin KF (an Escherichia coli K12 strain MG1655) were ligated to construct a KFD gene (SEQ ID NO. 21), which was then cloned into a pET-28a plasmid vector (Invitrogen). A pET-28a-KFD recombinant plasmid was transformed into an Escherichia coli BL21 DE3 strain, and the transformed bacteria were cultured in Luria-Bertani broth containing 50 mg/ml kanamycin at 37°C overnight. For expression of KFD, bacteria in a logarithmic phase were induced with IPTG plus lactose; and in order to increase the yield of soluble protein, 1% ethanol was added 1 hour before induction, the culture temperature was reduced to 18°C, and the bacteria were grown for 16 hours, and harvested.

A scaffold NC was designed based on a 3D structure of KFD to preserve conserved domains D0 and D1. An N-polypeptide (SEQ ID NO. 1) and a C-polypeptide (SEQ ID NO. 3) have about 72% amino acid (aa.) homology to KFD (SEQ ID NO. 21). Polynucleotide sequences encoding an RBD (aa. 319-527) of an Omicron variant BA.1 (B.1.1.529) of SARS-CoV-2 (SEQ ID NO. 8), an N-polypeptide (SEQ ID NO. 2), an RBD (aa. 319-527) of a Delta variant (SEQ ID NO. 6), a C-polypeptide (SEQ ID NO. 4) and an RBD (aa. 319-527) of an Omicron variant (SEQ ID NO. 8) were sequentially ligated to construct a 3Ro-NC gene (SEQ ID NO. 18). The polynucleotide sequences encoding the RBD (aa. 319-527) of the Omicron variant BA.1 (B.1.1.529) of SARS-CoV-2 (SEQ ID NO. 8), an N-polypeptide (SEQ ID NO. 2), an RBD (aa. 319-527) of an SHC014 variant (SEQ ID NO. 14), a C-polypeptide (SEQ ID NO. 4) and an RBD (aa. 319-527) of a WIV1 variant (SEQ ID NO. 16) were sequentially ligated to construct a 3Rs-NC gene (SEQ ID NO. 20). The polynucleotide sequences encoding two RBDs (aa. 319-527) from a SARS-CoV-2 original strain (SEQ ID NO. 10) were ligated to construct an RBD dimer gene. In the presence of a signal peptide tPA in a 5' region, genes encoding RBDs (aa. 319-527) of the SARS-CoV-2 original strain, the Gamma variant (SEQ ID NO. 12), the Delta variant (Rd), and the Omicron variant (Ro), the RBD dimer gene, as well as the 3Ro-NC gene were cloned into a pcDNA 3.1 plasmid vector, respectively. 293F cells obtained from Thermo Fisher Scientific were transfected with a recombinant plasmid in the presence of polyethylenimine (PEI). The culture supernatant was collected after incubation at 37°C and 5% CO₂ under shaking at 130 rpm for 72 h.

For each construct, a 6×His tag or an 8×His tag was added to a C-terminus to facilitate protein purification. The recombinant proteins were purified by affinity chromatography on a Ni-NTA column (QIAGEN) and contaminated lipopolysaccharides (LPSs) were removed respectively. The residual LPS content was determined by using a Limulus assay (a Cape Cod complex), and was less than 0.01 EU/µg protein.

### 1.3. Caco-2 cells, culture and determination of TLR5 agonist activity

Caco-2 cells (HTB-37) obtained from the American Type Culture Collection (ATCC) were used to test the TLR5 agonist activity. The Caco-2 cells were maintained in a Dulbecco's Modified Eagle's Medium (DMEM) supplemented with 10% (v/v) fetal bovine serum (FBS) and 1% (v/v) penicillin/streptomycin (a standard medium) to be cultured at 37°C and 5% carbon dioxide. The cells were seeded into a 24-well plate at 2×10⁵/well, and maintained in a standard medium for 5 days at 37°C and 5% CO₂ to form a tight cell monolayer, and then cultured overnight in a serum-free DMEM, followed by stimulation with a continuous concentration gradient of recombinant protein for 8 hours; the supernatants were collected for detection of interleukin-8 (IL-8) by an enzyme-linked immunosorbent assay (ELISA) kit (BD Bioscience).

### 1.4. Preparation of SARS-CoV-2 inactivated vaccine (IAV)

SARS-CoV-2 virus (an original strain) was used to infect Vero cells. The supernatant was collected on day 3 or day 4 when cytopathic effects (CPEs) were observed. Beta-propiolactone was then added to the supernatant in a ratio of 1:4000 (v/v), and the virus was inactivated at 2°C-8°C for 48 h, followed by removal of cell debris and concentration by ultrafiltration. Inactivation was verified by passaging the treated sample for 3 passages without the appearance of CPE. After gel chromatography, ion exchange chromatography, and sterile filtration, the viral particles were formulated with buffer.

### 1.5. Vaccination

6- to 8-week-old female BALB/c or 12- to 16-week-old hACE2 mice were subjected to intramuscular injection immunization after mixing with Imject^{™} (Thermo Fisher) (AL-adjuvant) in a lower hind limb or intranasal immunization after mixing with a flagellin-derived KFD protein adjuvant for three times at three-week intervals. Anesthesia was performed with pentobarbital sodium (50 mg/kg) before intranasal immunization.

### 1.6. Schematic diagram of binding of SARS-CoV-2 RBD to neutralizing antibodies

The simulated structures of complexes of a receptor binding domain of SARS-CoV-2 and neutralizing antibodies B38, COV2-39, CR3022 and REGN10987 were downloaded from a PDB database (PDB ID:7BZ5, 7JMP, 6W41, and 6XDG) and imported into software Pymol, respectively. Four RBDs were docked together to create a 3D structure of SARS-CoV-2 RBD bound by four different neutralizing antibodies.

### 1.7. Enzyme-linked immunosorbent assay (ELISA)

Antibody responses were assessed by ELISA. Briefly, a 96-well plate was coated with a target protein (2 µg/mL) in a carbonate-bicarbonate buffer at 4°C overnight, and then blocking was performed with 1% BSA for 2 hours at 37°C. Four-fold serially diluted samples were then added to wells, and incubated at 37°C for 2 hours. After washing, an alkaline phosphatase-labeled secondary antibody (goat anti-mouse IgG, human ads-AP antibody or goat anti-mouse IgA-AP antibody, SouthernBiotech) was added to wells, followed by substrate (p-nitrophenyl phosphate, Sigma) coloring after washing. OD values were read by an ELISA plate reader (Thermo Labsystems) at 405 nm.

### 1.8. Pseudovirus preparation and neutralization assay

To prepare SARS-CoV-2 spike pseudoviruses, 60 µg of plasmid pNL4-3.luc.RE and 20 µg of plasmid expressing spike of different SARS-CoV-2 variants were co-transfected with PEI into a 15 cm cell culture dish in which HEK293T cells were cultured. The supernatant was collected 72 hours after transfection, centrifuged at 1000 rpm, and stored at -80°C for later use. To assess the neutralization efficiency of serum, samples were serially diluted from 1: 10 to 1:3000 with DMEM supplemented with 10% FBS, with a total volume of 50 µl, and then co-incubated with 20 µl of 200×TCID₅₀ SARS-CoV-2 pseudovirus for 1 hour at 37°C. 30 µl of a complete medium containing approximately 3×10⁵ ACE2-293T cells was added into each well, and incubation was performed for 48 hours at 37°C and 5% CO₂. Luciferase activity was analyzed by a Luciferase Assay System (Promega). Inhibition of the SARS-CoV-2 pseudovirus was expressed as percent inhibition. The 50% neutralization titer (NT50) was determined by four-parameter logistic regression analysis using GraphPad Prism 8.0 (GraphPad Software Inc.).

### 1.9. Antigen cartography

Antigen cartography was created with R packages Racmacs (https://acorg.github.io/Racmacs/index.html) by using serum neutralization titers against SARS-CoV-2 pseudovirus (Alpha, Beta, Gamma, Delta, and Omicron variants and a Wuhan-Hu-1 strain) and SARS-CoV-1 pseudovirus. The antigenic distance was measured in antigenic units (AU). One AU corresponds to a two-fold dilution of antibody in a neutralization assay. Each square in the cartography represents 1 AU. The antigenic distance can be measured in any direction of the cartography.

### 1.10. Preparation and inoculation of Omicron variant of SARS-CoV-2

The Omicron strain BA.1 of SARS-CoV-2 (IVCAS6.7600) was provided by the National Virus Resource Center (Wuhan, China). Viruses were propagated and titrated in Cercopithecus aethiops kidney cells (Vero-E6, ATCC CRL-1586). For the vaccine protection experiments, 28 days after the last immunization, HFH4-hACE2 mice were inoculated intranasally with 50 µl of the Omicron strain of SARS-CoV-2 (5×10⁴ TCID₅₀) under avertin (250 mg/kg) anesthesia. At 3 days post-infection, the mice were euthanized and then the lung and nasal turbinate tissues were harvested. All procedures involving infectious SARS-CoV-2 were performed at a biosafety level 3 laboratory.

### 1.11. Tissue collection and virus titration

Vero-E6 cells were seeded into a 24-well plate 1 day before use. Infected lungs and nasal turbinates were homogenized in DMEM and 10-fold serially diluted. The cells were seeded with a tissue dilution buffer for 1 hour. Next, an inoculum was removed, and the cells were incubated with 0.9% methylcellulose for 5 days. Plaques were counted after crystal violet staining and virus titers were calculated.

### 1.12. Histology

Mouse lungs were excised and fixed with 4% paraformaldehyde for 1 week at room temperature, and then embedded with paraffin. After sectioning, tissue sections were used for hematoxylin and eosin (H&E) staining. Whole slice imaging was observed by using a slice scanner Pannoramic MIDI (3DHISTECH). Pathological changes were assessed and scored on a 1-4 severity scale. Scoring criteria for inflammatory cell aggregation and interstitial pneumonia: around the air gaps of bronchioles or pulmonary vessels or lung sections, 1-normal, 2-mild and occasional cell aggregation, 3-moderate cell infiltration, and 4-moderate to severe and multifocal cell aggregation.

### 1.13. Statistical analysis

The mean, SD, neutralization titer and correlation were calculated by using GraphPad Prism 8.0 software. A significance test was applied as shown in a legend for each figure. Statistical analysis was performed by using one-way ANOVA and a Dunnett's multiple comparison test unless otherwise indicated. To analyze differences between two groups, an unpaired two-tailed Student's t-test was used for normally distributed data with equal variance and a Mann-Whitney U-test was used for non-normally distributed data. Simple linear regression was used for correlation analysis. Analysis was performed by using GraphPad Prism 8.0 software. Significance values were expressed as *P<0.05, **P<0.01 and ***P<0.001; ns, not significant. P<0.05 was considered significant.

### 2. Experimental results

### 2.1. Chimeric triple RBD protein 3Ro-NC retains neutralizing epitopes

FIG. 1 shows a schematic domain configuration of KF, KFD and a chimeric triple RBD protein called 3Ro-NC. As shown in FIG. 1, in 3Ro-NC, three immunodominant RBDs from SARS-CoV-2 variants of concern (VOC) Omicron and Delta were integrated into NC. The chimeric protein 3Ro-NC includes: NC, one RBD of the Delta variant integrated at the N-polypeptide and C-polypeptide junction of NC, and two RBDs of the Omicron variant BA.1 (B.1. 1.529) linked to the N-terminal and C-terminal of NC, respectively.

FIG. 2 shows a 3D structure of 3Ro-NC predicted by Alpha Fold 2. As shown in FIG. 2, three RBDs are presented in a stabilized conformation.

FIG. 3 is a Western blot image of purified 3Ro-NC. The immunogen 3Ro-NC was expressed in 293F cells and purified as a single band with a molecular weight of approximately 120 kDa and a purity of over 95% as verified by sodium dodecyl sulfate-polyacrylamide gel electrophoresis (SDS-PAGE).

FIG. 4 shows the TLR5 agonist activity of recombinant proteins such as RBD of a SARS-CoV-2 original strain, RBD of Omicron, P-KFD, and 3Ro-NC. Recombinant protein-stimulated IL-8 production in the supernatant of cultured Caco-2 cells was detected by ELISA, 3 replicates per group. P-KFD is a flagellin fusion protein expressed in our previously published Escherichia coli system (B. Zhao et al., A safe and effective mucosal RSV vaccine in mice considering of RSV phosphoprotein and flagellin variant. Cell reports 36, 109401 (2021).). Unlike the previously generated Escherichia coli system expressing the flagellin fusion protein P-KFD (7), 3Ro-NC has no toll-like receptor 5 (TLR5) activating activity.

FIG. 5 shows a 3D schematic diagram of the binding of SARS-CoV-2 RBD to four classes of neutralizing monoclonal antibodies. Four monoclonal antibodies (mAb) B38, M-S309, CR3022 and COVA2-39 represent four classes of RBD-specific neutralizing antibodies (B. He et al., Rapid isolation and immune profiling of SARS-CoV-2 specific memory B cell in convalescent COVID-19 patients via LIBRA-seq. Signal transduction and targeted therapy 6, 195 (2021).).

FIG. 6 is a graph showing three ELISA results, showing the binding ability of RBD of a Delta strain (Rd), RBD of an Omicron (B. 1.1.529) BA. 1 strain (Ro) and 3Ro-NC to four representative classes of neutralizing monoclonal antibodies B38, M-S309, CR3022 and COVA2-39. As shown in FIG. 6, although the monomeric RBD of the Delta variant (Rd) showed potent binding ability to all four monoclonal antibodies, the monomeric RBD of the Omicron variant (Ro) had significantly reduced binding ability to mAb CR3022 and M-S309 and eliminated binding ability to B38 and COVA2-39. In contrast, the binding ability of 3Ro-NC to all four mAbs was high, even with higher affinity than Rd, indicating that a satisfactory native conformation of RBD is retained in the chimeric protein 3Ro-NC.

FIG. 7 shows the immunization and sampling schedule (5 mice per group). To analyze the immunogenicity of 3Ro-NC, BALB/c mice were immunized by intramuscular injection with 3Ro-NC or the RBD dimer of the SARS-CoV-2 original strain (5. L. Dai et al., A Universal Design of Betacoronavirus Vaccines against COVID-19, MERS, and SARS. Cell 182, 722-733.e711 (2020).) and an aluminum adjuvant (AL-adjuvant) at 4 µg/dose for three times. Saline alone was administered as a control. Mouse serum samples were collected 14 days after each immunization and humoral responses were detected by using ELISA.

FIG. 8 is a graph showing RBD-specific IgG responses in serum. After the 1st and 2nd immunizations, 3Ro-NC (3Ro-NC+AL i.m) elicited relatively higher RBD-specific IgG responses not only against the RBDs of the Omicron and Delta variants, but also against the RBDs of the Gamma and original strains of SARS-CoV-2 compared with the RBD dimer (RBD dimer+AL i.m). Notably, only 3Ro-NC induced prominent Omicron RBD-specific IgG responses after the 1st immunization.

FIG. 9 is a dot plot showing the results of titers of neutralizing antibodies against SARS-CoV-2 variants after the 2nd and 3rd immunizations in a pseudovirus assay; neutralizing antibodies against SARS-CoV-2 VOC were assessed by the pseudovirus assay. Sera from 3Ro-NC immunized mice (3Ro-NC+AL i.m) could effectively neutralize pseudoviruses of all tested SARS-CoV-2 variants, with higher neutralization titers than those of RBD dimer immunized mice (RBD dimer+AL i.m) after the 2nd and 3rd immunizations, although not significant at all time points.

FIG. 10 is a dot plot showing the results of titers of neutralizing antibodies against SARS-CoV-1 after the 2nd and 3rd immunizations in a pseudovirus assay; sera from 3Ro-NC immunized mice (3Ro-NC+AL i.m) could effectively neutralize SARS-CoV-1 pseudovirus, with higher neutralization titers than those of RBD dimer immunized mice (RBD dimer+AL i.m) after the 2nd and 3rd immunizations, although not significant at all time points.

FIG. 11 is a dot plot showing the results of titers of neutralizing antibodies against Omicron and the original strain after the 1st immunization in a pseudovirus assay; notably, after the 1st immunization, only 3Ro-NC could induce prominent neutralizing antibodies against Omicron and the original strain.

FIG. 12 is a graph showing a geometric mean titer (GMT) of neutralizing antibodies against different variants. The geometric mean titer of the neutralizing antibodies also indicated that 3Ro-NC could induce potent neutralizing antibody responses against all tested SARS-CoV-2 variants as well as SARS-CoV-1.

FIG. 13 is a dot plot showing a ratio of the titer (a 50% neutralization titer, NT50) of neutralizing antibodies against Omicron to the titer of neutralizing antibodies against the original strain or Delta; in 3Ro-NC immunized mice, the ratio of the neutralization titer of the Omicron variant to the neutralization titer of the original strain or the Delta variant was significantly higher than that in RBD dimmer immunized mice (6.1-fold and 10.7-fold, respectively, post 3rd immunization), suggesting that the antibody response induced by 3Ro-NC can easily neutralize Omicron, which has the potential to evade neutralization.

FIG. 14 shows the antigenic map generated from serum samples post 2nd (squares) or 3rd (triangles) vaccination in RBD dimer (white) and 3Ro-NC (grey) groups. We also applied antigenic cartography to explore how serum samples distinguish different spike antigens. After the 2nd or 3rd immunization, neutralizing antibody titers were used to make antigenic maps, respectively. For the RBD dimer+AL i.m group, serum samples post 2nd and 3rd vaccinations (FIG. 14, an upper panel) were clustered more tightly around the original strain and the Delta strain. Consistent with neutralization titers (FIGS. 9, 11, 12 and 13), the antigenic distance between Omicron and the original strain or Delta was large for these two groups of RBD dimer immune sera. For the 3Ro-NC+AL i.m group, although the serum samples post 2nd vaccination (FIG. 14, a lower left panel) were still tightly clustered around the original strain and the Delta strain, the antigenic distance between the Omicron and the original strain (a 9.2-fold difference) was closer than that in the RBD dimer+AL i.m group (a 25.1-fold difference). Notably, the distance between Omicron and Delta was significantly reduced from a 26.9-fold difference to a 3.5-fold difference after the 3rd 3Ro-NC vaccination (FIG. 14, a lower panel). More notably, post 3rd vaccination, the antigenic distance between Omicron and the original strain or Delta in the 3Ro-NC+AL i.m group (6.1-fold and 3.5-fold, respectively) was closer than that in the RBD dimer+AL i.m group (55.7-fold and 48.5-fold differences) (FIG. 14, a right panel). The change in antigenic distance to Omicron indicates that an increase in the proportion of neutralizing antibodies against Omicron occurred after the 3rd immunization in the 3Ro-NC+AL i.m group. These results indicate that 3Ro-NC has extraordinary immunogenicity and can induce effective and broad-spectrum RBD-specific IgG responses against SARS-CoV-2 variants, even SARS-CoV-1, particularly for the highly immune escape Omicron variant.

FIG. 15 shows the immunization and sampling schedule to test antibody responses generated by intranasal immunization with 3Ro-NC plus KFD adjuvant in BALB/c mice (n=5 mice per group). In developing an effective mucosal vaccine, a suitable mucosal adjuvant is often essential to a subunit protein immunogen. Briefly, BALB/c mice were subjected to intranasal immunization with 4 µg 3Ro-NC plus 1 µg KFD adjuvant (3Ro-NC+KFD i.n) or intramuscular injection immunization with 4 µg 3Ro-NC plus 200 µg AL-adjuvant (3Ro-NC+AL i.m) for 3 times. Saline alone was administered as a control. Mouse serum and mucosal samples were collected 14 days after each immunization, and humoral responses were detected by using ELISA.

FIG. 16 is a dot plot showing the results of Delta, Gamma or Omicron RBD-specific IgG responses in serum. The results showed that after the 2nd and 3rd immunizations, the level of RBD-specific IgG responses induced by the 3Ro-NC+KFD i.n immunization group was similar to that in the 3Ro-NC+AL i.m group, although much lower antibody titers were observed in the 3Ro-NC+KFD i.n group after the 1st immunization.

FIG. 17 is a graph showing titers of neutralizing antibodies against SARS-CoV-2 variants or SARS-CoV-1 by a pseudovirus assay in serum after the 2nd and 3rd immunizations in 3Ro-NC+KFD intranasal immunization and 3Ro-NC+AL intramuscular injection groups, wherein each connecting line represents the neutralization titer of one single serum sample to a different viral strain. Serum neutralizing antibodies were assessed with pseudoviruses. In general, after the 2nd and 3rd immunizations, sera from 3Ro-NC+KFD i.n immunized mice could effectively neutralize all tested SARS-CoV-2 variants, even SARS-CoV-1, although the neutralization titers for most variants were slightly lower than those of 3Ro-NC+AL i.m immunized mice. It should be noted that neutralization titers against the Omicron variant were comparable to those of the Delta variant and even higher than those of the original strain only in the 3Ro-NC+KFD i.n immunized mice.

FIG. 18 is a graph showing the geometric mean titer of neutralizing antibodies against different variants in serum after the 2nd and 3rd immunizations in 3Ro-NC+KFD intranasal immunization and 3Ro-NC+AL intramuscular injection groups. Interestingly, the geometric mean titer (GMT) of neutralizing antibodies against Omicron in the sera of 3Ro-NC+KFD i.n immunized mice was even higher than that of 3Ro-NC+AL i.m immunized mice, especially at the time point after the 2nd immunization.

FIG. 19 is a dot plot showing a ratio of the titer of neutralizing antibodies against Omicron to the titer of neutralizing antibodies against Delta or the original strain. In 3Ro-NC+KFD i.n immunized mice, the ratio of the neutralization titer of the Omicron variant to the neutralization titer of the original strain or the Delta variant was significantly higher than that in 3Ro-NC+AL i.m immunized mice (42-fold and 32-fold, respectively, post 2nd immunization).

FIG. 20 shows an antigenic map generated from serum samples after the 2nd (squares) or 3rd (triangles) vaccination in the 3Ro-NC+KFD intranasal immunization group; FIG. 21 shows an antigenic map generated by the 3Ro-NC+KFD intranasal immunization group (brown) and the 3Ro-NC+AL intramuscular injection group (grey); antigenic cartography is used to explore how sera from 3Ro-NC intranasally immunized mice distinguish different spike antigens. Antigenic maps were made by using neutralizing antibody titers alone or together after the 2nd or 3rd immunization (FIG. 20 and FIG. 21). For the 3Ro-NC+KFD i.n group, the serum samples after the 2nd and 3rd vaccinations were both tightly clustered around the Delta and Omicron strains (FIG. 20 and FIG. 21), while the serum samples in the 3Ro-NC+AL i.m group were all tightly clustered around the Delta strain (FIG. 21). After the 2nd vaccination, the antigenic distance between Omicron and Delta in the 3Ro-NC+KFD i.n group (a 6.1-fold difference) was closer than that in the 3Ro-NC+AL i.m group (a 26.9-fold difference) (FIG. 20, a left panel). After the 3rd vaccination, the antigenic distance between Omicron and the original strain or Delta in the 3Ro-NC+KFD i.n group (2.8-fold and 1.7-fold differences, respectively) was still closer than that in the 3Ro-NC+AL i.m group (6.1-fold and 3.5-fold differences, respectively) (FIG. 20, a right panel). The difference in antigenic distance from Omicron reflects that 3Ro-NC+KFD i.n immunization tends to induce stronger neutralizing antibody responses against the Omicron variant compared with 3Ro-NC+AL i.m immunization, indicating that changes in the immunization route and adjuvant type significantly alter the specificity of the antibody response.

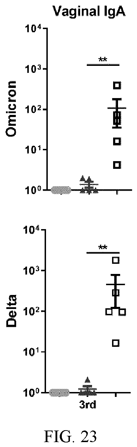

FIG. 22, FIG. 23 and FIG. 24 are dot plots showing RBD-specific mucosal IgA responses in saliva, vaginal lavage fluid, and nasal turbinate lavage fluid, respectively. During mucosal immune response evaluation, as expected, only intranasal immunization (3Ro-NC+KFD i.n) could induce specific mucosal IgA antibody responses against Omicron and Delta RBD in the saliva (FIG. 22). In addition, similar levels of IgA responses were also detected in the vaginal lavage fluid (FIG. 23) and nasal lavage fluid (FIG. 24, a right panel) after the 3rd intranasal immunization. When comparing different vaccination routes, similar titers of RBD-specific IgG (about 102) could be detected in the nasal lavage fluid of intramuscularly (3Ro-NC+AL i.m) and intranasally (3Ro-NC + KFD i.n) immunized mice (FIG. 24, a left panel).

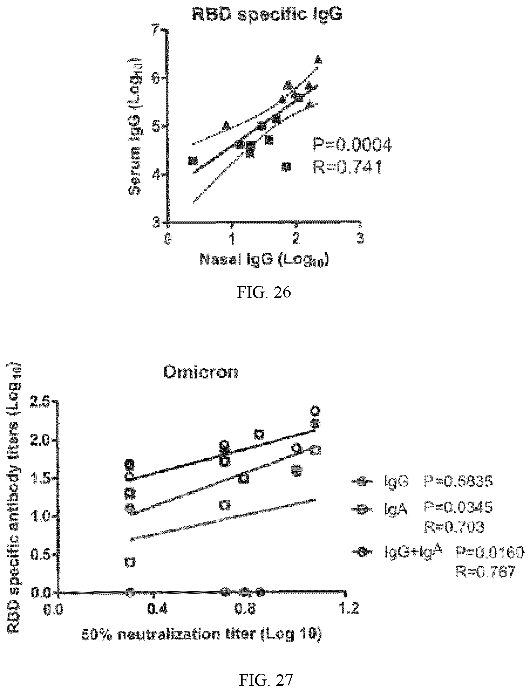

FIG. 25, FIG. 26 and FIG. 27 show the correlation between RBD-specific IgA in nasal lavage fluid and RBD-specific IgA in saliva (triangles represent Delta; and squares represent Omicron), the correlation between RBD-specific IgG in nasal lavage fluid and RBD-specific IgG in serum (triangles represent Delta; and squares represent Omicron), and the correlation between RBD-specific IgA and IgG in nasal lavage fluid and neutralizing antibody responses of BALB/c mice immunized with 3Ro-NC determined in a pseudovirus system, respectively. A high correlation between RBD-specific IgA titers in the nasal lavage fluid and titers in the saliva can be observed (FIG. 25), suggesting that salivary IgA responses may reflect IgA responses in the upper respiratory tract. This lower titer of detectable RBD-specific mucosal IgG was shown to be highly correlated with a higher titer of RBD-specific serum IgG (FIG. 26). Interestingly, serum neutralization titers of Omicron showed significant correlation with Omicron RBD-specific nasal IgA responses or nasal IgA plus IgG levels, but not with nasal IgG responses (FIG. 27). These data suggest that in nasal mucosas, RBD-specific antibodies, especially mucosal IgA, may play a key role in blocking initial SARS-CoV-2 infection.

Overall, 3Ro-NC has high immunogenicity through intranasal immunization with KFD as the adjuvant and can induce coordinated systemic and local mucosal immune responses against different SARS-CoV-2 variants, particularly against Omicron.

FIG. 28 shows the immunization and viral challenge schedule used to investigate the protection of 3Ro-NC immunized hACE2 mice against infection with the Omicron variant of SARS-CoV-2 (6-8 mice per group). To further explore the protective efficacy of the 3Ro-NC vaccine, human ACE2 transgenic mice (hACE2) were utilized to evaluate mucosal protection against infection with the Omicron variant of SARS-CoV-2. Briefly, immunization with 3Ro-NC plus KFD by an intranasal route (3Ro-NC+KFD i.n), or 3Ro-NC plus the AL-adjuvant by an intramuscular route (3Ro-NC+AL i.m), or 2.5 µg of inactivated SARS-CoV-2 plus 200 µg of the AL adjuvant by the intramuscular route (IAV+AL i.m) was performed, and inoculation using saline was used as a control group.

FIG. 29, FIG. 30 and FIG. 31 are dot plots showing Omicron, Delta and Gamma RBD-specific serum IgG titers; Delta and Omicron RBD-specific salivary IgA; and Delta and Omicron RBD-specific vaginal lavage fluid IgA after the 3rd immunization, respectively; consistent with the results in BALB/c mice, strong serum IgG antibody responses against Omicron, Delta and Gamma RBD were induced in the mice by intranasal immunization (3Ro-NC+KFD i.n) and intramuscular injection immunization (3Ro-NC+AL i.m). Among the three immunization groups, the IAV+AL i.m group produced the lowest RBD-specific serum IgG, while the 3Ro-NC+KFD i.n group produced the highest Omicron RBD-specific serum IgG (FIG. 29). RBD-specific IgA could only be induced in the mice of the 3Ro-NC+KFD i.n group in the saliva (FIG. 30) and the vaginal lavage fluid (FIG. 31).

FIG. 32 is a dot plot showing the RNA copy number of the SARS-CoV-2 RBD gene in nasal turbinates and lungs tested by qPCR, and a plaque assay of infectious virus in lungs 3 days post-infection, respectively. Twenty-eight days after the 3rd immunization, mice were challenged intranasally with 5×10⁴ TCID₅₀ Omicron variant in a total volume of 50 µl. After three days of exposure to the Omicron variant, viral loads in nasal turbinate tissue and lungs were determined by using qPCR and the plaque assay. Only the 3Ro-NC+KFD i.n group showed a significant reduction in virus in the nasal turbinates compared with the saline group or the other two intramuscular injection immunization groups. In lung tissues, both the virus copy number in the IAV+AL i.m group and the virus copy number in the 3Ro-NC+KFD i.n group were significantly reduced (FIG. 32, a middle panel). In the 3Ro-NC+AL i.m group, only part of the mice showed a decrease in Omicron viral load in lung tissues, although Omicron RBD-specific serum IgG titers in this group were even higher than those in the IAV+AL i.m group. Compared with the saline control group, the infectious virus was almost undetectable in the lung tissues of all three immunization groups except for one mouse in the 3Ro-NC+AL i.m group (FIG. 32, a right panel).

FIG. 33 shows images of hematoxylin and eosin (H&E) staining of lung sections (scale, 100 µm). FIG. 34 is a dot plot showing pathology scores and infiltration scores of immune cell aggregation around bronchioles, pulmonary vessels and interstitial pneumonia according to H&E stained sections (6-8 mice/group); simultaneously, histopathological examination was performed to analyze infection- and immunization-related inflammation in the lungs after viral challenge (FIG. 33). The results showed that widely distributed inflammatory cell infiltration could be observed in the lungs of IAV-immunized (IAV+AL i.m) mice, especially at perivascular sites (FIG. 33). In contrast, inflammatory cell infiltration in the lungs of mice immunized with 3Ro-NC+KFD i.n or 3Ro-NC+AL i.m. was significantly reduced. These results indicate that intranasal immunization with 3Ro-NC plus the KFD adjuvant can restrict lung infection and pathology, suggesting that 3Ro-NC plus KFD as a prophylactic mucosal SARS-CoV-2 vaccine can provide protection against Omicron infection in both the upper and lower respiratory tracts.

FIG. 35 is a graph showing the correlation between the RNA copy number in lungs and the RNA copy number in nasal turbinates. In all vaccination groups, protection against Omicron infection in the alveolar or vascular parts could be more or less observed (FIG. 35). However, protection against infection in the lungs and nasal turbinates (FIG. 32) could only be observed in mice that had received intranasal immunization, which produced protective serum IgG and mucosal IgA. This raises the question that whether mucosal IgA and IgG exert different protective effects in the upper and lower respiratory tracts. Accordingly, in the experiment, no significant correlation in virus copy number between lung tissues and nasal turbinates was observed (FIG. 36). Due to the lack of nasal or bronchoalveolar lavage fluid before the sacrifice of the mice, we chose salivary IgA and serum IgG, which are highly correlated with nasal IgA and IgG levels, respectively, as representatives of humoral immune responses. In lung tissues, the copy number of viral RNAs was negatively correlated with serum IgG titers, but not with salivary IgA titers (FIG. 36). On the other hand, the copy number of viral RNAs in nasal turbinate tissue was only negatively correlated with salivary IgA titers, but not with serum IgG titers (FIG. 37), indicating that protection of the lungs and nasal turbinates is provided primarily by RBD-specific IgG responses and mucosal IgA, respectively.

FIG. 38 is a dot plot showing serum RBD-specific IgG responses after the 2nd immunization; and FIG. 39 is a dot plot showing titers of neutralizing antibodies against the SARS-CoV-2 variant Omicron or SARS-like viruses SHC014 and WIV1 after the 2nd immunization.

The above are only the preferred examples of the present invention and are not intended to limit the present invention in any form or substance. It should be pointed out that those of ordinary skill in the art can make some improvements and supplements without departing from the present invention, and these improvements and supplements should also be regarded as the scope of protection of the present invention. All equivalent changes of some changes, modifications, and evolutions made by those skilled in the art by utilizing the technical contents disclosed above without departing from the spirit and scope of the present invention are equivalent examples of the present invention; meanwhile, any equivalent changes, modifications, and evolutions made to the above examples in accordance with the substantive technical aspects of the present invention are still within the scope of the technical solutions of the present invention.

## Claims

1. A recombinant multivalent vaccine, comprising a recombinant protein, **characterized in that** the recombinant protein comprises, from a N-terminus to a C-terminus, a first antigenic peptide, an N-polypeptide, a second antigenic peptide, a C-polypeptide and a third antigenic peptide;
wherein the N-polypeptide has an amino acid sequence as shown in SEQ ID NO. 1 or a variant thereof;
the C-polypeptide has an amino acid sequence as shown in SEQ ID NO. 3 or a variant thereof; and
the N-polypeptide and the C-polypeptide are intramolecular scaffold-forming polypeptides, forming an intramolecular scaffold NC for supporting and stabilizing conformations of the first antigenic peptide, the second antigenic peptide and the third antigenic peptide.

2. The recombinant multivalent vaccine according to claim 1, **characterized in that** the variant of the N-polypeptide is functionally identical to and has at least 95% sequence homology with SEQ ID NO. 1; and the variant of the C-polypeptide is functionally identical to and has at least 95% sequence homology with SEQ ID NO. 3.

3. The recombinant multivalent vaccine according to claim 1, **characterized in that** the first antigenic peptide, the second antigenic peptide and the third antigenic peptide are polypeptides consisting of 10-900 amino acids that induce an antigenic peptide specific humoral and/or cellular immune response when the recombinant multivalent vaccine is used to immunize a host subject.

4. The recombinant multivalent vaccine according to claim 1, **characterized in that** the first antigenic peptide, the second antigenic peptide and the third antigenic peptide are derived from an infectious pathogen, a variant of an infectious pathogen or a cancer/tumor-specific antigen.

5. The recombinant multivalent vaccine according to claim 1, **characterized in that** the first antigenic peptide, the second antigenic peptide and the third antigenic peptide are derived from a SARS-CoV-2 variant.

6. The recombinant multivalent vaccine according to claim 5, **characterized in that** the antigenic peptide is a receptor binding domain (RBD) region aa.319-527 of a spike protein of the SARS-CoV-2 variant.

7. The recombinant multivalent vaccine according to claim 6, **characterized in that** the RBD is derived from any one of a SARS-CoV-2 original strain, Delta, Omicron, and Gamma variants and SARS-related coronavirus strains such as SHC014 and WIV1, wherein Delta RBD has an amino acid sequence as shown in SEQ ID NO. 5, Omicron RBD has an amino acid sequence as shown in SEQ ID NO. 7, RBD of the SARS-CoV-2 original strain has an amino acid sequence as shown in SEQ ID NO. 9, Gamma RBD has an amino acid sequence as shown in SEQ ID NO. 11, SHC014 RBD has an amino acid sequence as shown in SEQ ID NO. 13, and WIV1 RBD has an amino acid sequence as shown in SEQ ID NO. 15.

8. The recombinant multivalent vaccine according to claim 1, **characterized in that** the recombinant protein comprises, from the N-terminus to the C-terminus, Omicron RBD, an N-polypeptide, Delta RBD, a C-polypeptide and Omicron RBD, having an amino acid sequence as shown in SEQ ID NO. 17; or the recombinant protein comprises, from the N-terminus to the C-terminus, Omicron RBD, an N-polypeptide, SHC014 RBD, a C-polypeptide, and WIV1 RBD, having an amino acid sequence as shown in SEQ ID NO. 19.

9. The recombinant multivalent vaccine according to claim 1, **characterized by** further comprising an adjuvant selected from at least one of flagellin, polyinosinic-polycytidylic acid, MF59, AS01, AS03, AS04, CpG, MPL, CT, CTB, IL-1α, IL-2, IL-12, IL-18, GM-CSF, PIKA, and BFA03.

10. The recombinant multivalent vaccine according to claim 9, **characterized in that** the adjuvant is recombinant flagellin KFD, having an amino acid sequence shown in SEQ ID NO. 21 or having at least 95% sequence homology with SEQ ID NO. 21 and retaining the TLR-5 agonist activity.

11. A nucleic acid molecule encoding a recombinant protein for a recombinant multivalent vaccine, **characterized in that** the nucleic acid molecule comprises from 5' to 3': a first polynucleotide encoding a first antigenic peptide, a polynucleotide encoding an N-polypeptide, a second polynucleotide encoding a second antigenic peptide, a polynucleotide encoding a C-polypeptide, and a third polynucleotide encoding a third antigenic peptide;
wherein the polynucleotide encoding the N-polypeptide has a nucleotide sequence shown in SEQ ID NO. 2 or a variant thereof;
the polynucleotide encoding the C-polypeptide has a nucleotide sequence shown in SEQ ID NO. 4 or a variant thereof; and
when a recombinant protein encoded by the nucleic acid molecule is expressed, the N-polypeptide and the C-polypeptide are intramolecular scaffold-forming polypeptides, forming an intramolecular scaffold NC for supporting the first antigenic peptide, the second antigenic peptide and the third antigenic peptide.
